(19) Europäisches Patentamt
European Patent Office
Office européen des brevets

(11) Veröffentlichungsnummer: **0 116 277**
**A2**

# (12) EUROPÄISCHE PATENTANMELDUNG

(21) Anmeldenummer: **84100139.9**

(22) Anmeldetag: **09.01.84**

(51) Int. Cl.³: **C 07 C 49/553**
C 07 C 45/28, C 07 C 45/58
C 07 C 45/54, C 07 C 143/68
C 07 C 35/36, C 11 B 9/00
A 61 K 7/46

(30) Priorität: **13.01.83 CH 189/83**

(43) Veröffentlichungstag der Anmeldung:
**22.08.84 Patentblatt 84/34**

(84) Benannte Vertragsstaaten:
**CH DE FR GB LI NL**

(71) Anmelder: **L. GIVAUDAN & CIE Société Anonyme**

**CH-1214 Vernier-Genève(CH)**

(72) Erfinder: **Klaus, Michael, Dr.**
**Am Hellenrain 6**
**D-7858 Weil/Rhein(DE)**

(72) Erfinder: **Helmlinger, Daniel, Dr.**
**Tichelrütistrasse 18**
**CH-8044 Gockhausen(CH)**

(72) Erfinder: **Pesaro, Mario, Dr.**
**Wiesliacher 55**
**CH-8053 Zürich(CH)**

(74) Vertreter: **Urech, Peter, Dr. et al,**
**Grenzacherstrasse 124 Postfach 3255**
**CH-4002 Basel(CH)**

(54) **Ketone von bicyclischen Verbindungen, Verfahren zu deren Herstellung und Riech- und/oder Geschmackstoffkompositionen mit einem Gehalt an solchen Verbindungen.**

(57) Die Erfindung betrifft neue Riech- und/oder Geschmackstoffe, nämlich Ketone der allgemeinen Formel

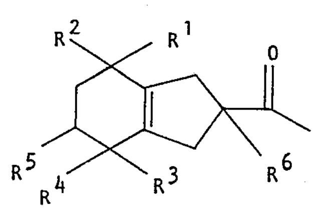

I

worin die Symbole $R^1$, $R^2$, $R^3$ und $R^4$ Wasserstoff, Methyl, Aethyl oder Isopropyl darstellen, wobei aber jeweils nich mehr als eines dieser Symbole für Wasserstoff, Aethyl oder Isopropyl steht, und die Symbole $R^5$ und $R^6$ Wasserstoff oder Methyl darstellen,

Die Erfindung betrifft auch Riech- und/oder Geschmackstoffkompositionen mit einem Gehalt an Verbindungen I, ein Verfahren zur Herstellung der Verbindungen I und die Verwendung der neuen Ketone I als Riech- und/oder Geschmackstoffe.



Croydon Printing Company Ltd.

L.Givaudan & Cie Société Anonyme, Vernier-Genève (Schweiz)



Ref. 6510/204

# Ketone von bicyclischen Verbindungen, Verfahren zu deren Herstellung und Riech-und/oder Geschmackstoffkompositionen mit einem Gehalt an solchen Verbindungen

Die Erfindung betrifft neue Riech- und/oder Geschmackstoffe. Es handelt sich dabei um die Verbindungen der allgemeinen Formel

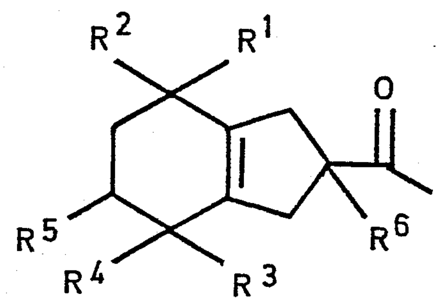

I

worin die Symbole $R^1$, $R^2$, $R^3$ und $R^4$ Wasserstoff, Methyl, Aethyl oder Isopropyl darstellen, wobei aber jeweils nicht mehr als eines dieser Symbole für Wasserstoff, Aethyl oder Isopropyl steht, und die Symbole $R^5$ und $R^6$ Wasserstoff oder Methyl darstellen.

Die Verbindungen I weisen besondere organoleptische Eigenschaften auf, auf Grund derer sie sich vorzüglich als Riech- und/oder Geschmackstoffe eignen.

Ur/ 19.10.83

Die Erfindung betrifft demgemäss auch die Verwendung der Verbindungen I als Riech- und/oder Geschmackstoffe und Riech- und/oder Geschmackstoffkompositionen mit einem Gehalt an Verbindungen I.

Die Erfindung betrifft auch ein Verfahren zu deren Herstellung. Dieses Verfahren ist dadurch gekennzeichnet, dass man

a) eine Verbindung der Formel

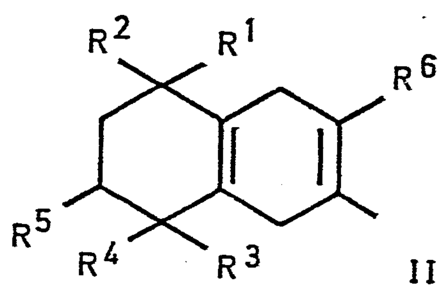

II

worin $R^1$ bis $R^5$ obige Bedeutung besitzen und $R^6$ Wasserstoff ist,
unter Ringkontraktion oxydativ umlagert, oder, dass man

b) eine Verbindung der Formel

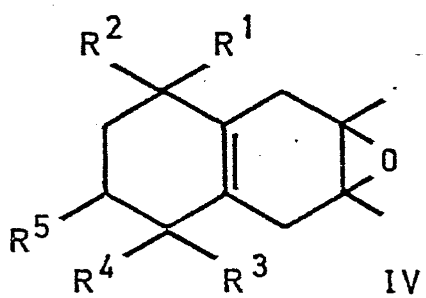

IV

worin $R^1$ bis $R^5$ obige Bedeutung besitzen,
isomerisiert, oder dass man

c) eine Verbindung der Formel

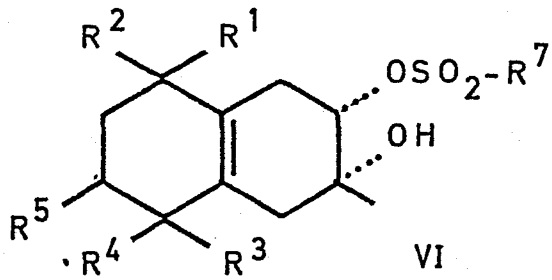

VI

worin $R^1$ bis $R^5$ obige Bedeutung besitzen und $R^7$ nieder-Alkyl oder Aryl ist,
unter Ringkontraktion isomerisiert.

Das folgende Reaktionsschema veranschaulicht diese drei Verfahrensvarianten als auch die Herstellung der Ausgangsmaterialien II, IV und VI.

Reaktionsschema I

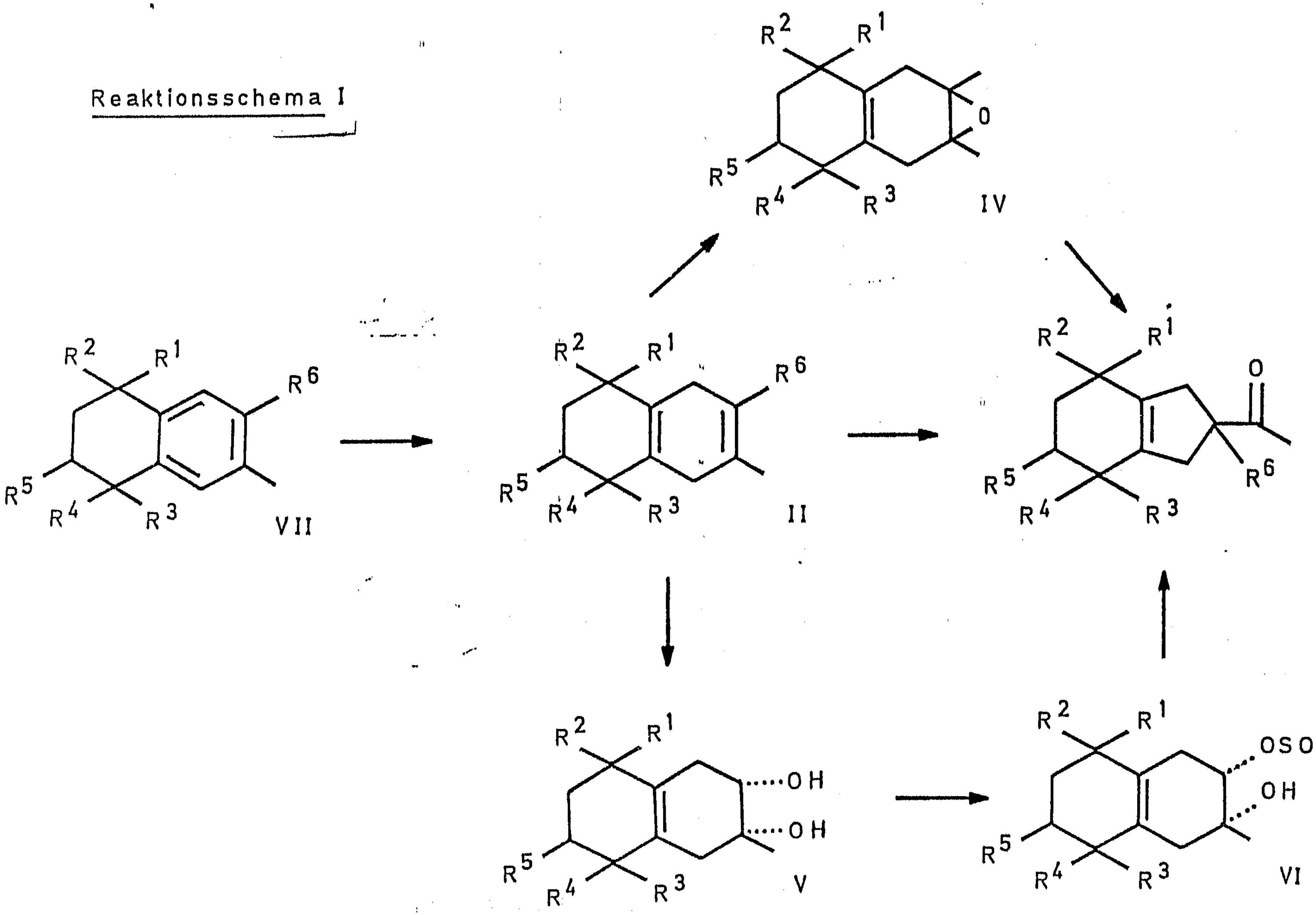

VII II IV I V VI

Das substituierte 6-Methyl-tetralin VII kann nach den an sich bekannten Methoden der Birch-Reduktion (vgl. z.B. A.J. Birch, G. Subba Rao, Advances in Organic Chemistry 8, 1 (1972)) oder der Reduktion nach Benkeser (vgl. z.B. R. Benkeser J. Org. Chem. 24, 854 (1959) und 29, 955, 1313 (1964)) zum bicyclischen 1,4-Dien der allgemeinen Formel II reduziert werden. Die Reduktion wird zweckmässigerweise mit einem Alkali- oder Erdalkalimetall, wie z.B. Lithium, Natrium, Calcium und Alkohol in flüssigem Ammoniak oder einem Alkylamin vorgenommen. Lösungsmittel wie z.B. Diäthyläther, Tetrahydrofuran oder Dimethoxyäthan können fakultativ verwendet werden. Ein Temperaturbereich von -40°C bis zum Siedepunkt des Amins ist für die Reduktion geeignet, wobei vorzugsweise 5-6°C unter dem Siedepunkt des Amins gearbeitet wird. Als Nebenprodukt der Reaktion entstehen die Tetrahydroverbindungen II'

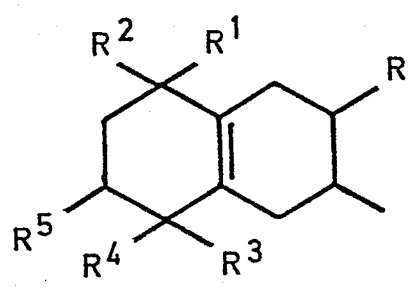

II'

die sich gegebenenfalls auf (gas)chromatographischem Weg abtrennen lassen. Für die weiteren Umsetzungen ist dies jedoch nicht nötig, da II' nicht störend in Erscheinung tritt. Vom wirtschaftlichen Gesichtspunkt aus verwendet man zweckmässigerweise das Gemisch II + II' als Ausgangsmaterial für das erfindungsgemässe Verfahren. Der direkte Zugang von II zu I (erfindungsgemässe Verfahrensvariante a)) kann beschritten werden, wenn $R^6$ Wasserstoff ist. Er beinhaltet die oxydative Umlagerung eines Olefins unter Ringkontraktion bei Verwendung von Thallium(III)-nitrat als Reagens (vgl. A. McKellop, Pure Appl. Chem. 43, 463 (1975)). Die Reaktion läuft unter schonenden Bedingungen

bei Raumtemperatur ab, wobei zweckmässigerweise in Alkoholen, insbesondere Methanol, als Lösungsmittel gearbeitet wird. Zur Aufarbeitung wird zunächst das Thallium(I)-salz abfiltriert und die Lösung anschliessend auf festes Natriumhydrogencarbonat gegossen. Die während der Reaktion gebildete Salpetersäure wird auf diese Weise neutralisiert. Nach kurzem, beispielsweise halbstündigem Rühren kann das Rohprodukt durch Abdestillieren des Alkohols (Methanol) und Destillation, z.B. fraktionierte Hochvakuumdestillation, auf einfache Weise weiter gereinigt werden. Als Nebenprodukt der Reaktion wird das Dimethylketal der allgemeinen Formel

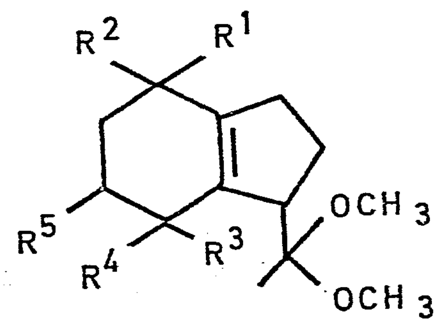

III

gebildet, das jedoch nicht aus dem Reaktionsgemisch abgetrennt werden muss, da es organoleptisch nicht störend in Erscheinung tritt. Eine Abtrennung ist jedoch möglich, z.B. durch Einsatz chromatographischer Methoden.

Im Falle von $R^6$ Methyl werden die bicyclischen 1,4-Diene II vorzugsweise in an sich bekannter Weise zu den bicyclischen Epoxiden der Formel IV umgesetzt. Verwendung finden können dabei alle gebräuchlichen Epoxidierungsmethoden, insbesonders diejenigen unter Verwendung von Persäuren, wie z.B. Peressigsäure, Metachlorperbenzoesäure usw. Die erfindungsgemässe Isomerisierung von IV zu den neuen Verbindungen I mit $R^6$ = Methyl (Verfahrensvariante b) verläuft vorzugsweise in Gegenwart von sauren Katalysatoren, insbesonders Lewis-Säure-Katalysatoren, wie z.B. Bortrifluorid-ätherat. Die bei Raumtemperatur einsetzende Reaktion führt zu einer Erwärmung des Reaktionsgemisches,

so dass zweckmässigerweise mit Aussenkühlung gearbeitet wird, die gestattet, die Temperatur in der Reaktionsmasse auf maximal 50°C zu halten. Die Umsetzung ist nach kurzer Zeit, z.B. nach 30 Minuten, beendet, und es kann wie üblich aufgearbeitet werden.

Bei der Oxidation von II, wobei $R^6$ Wasserstoff ist, zu den cis-1,2-Diolen V können z.B. Trimethylamin-N-oxid, N-Methylmorpholinoxid, Wasserstoffsuperoxid, tertiär-Butylhydroperoxid/Tetramethylammoniumacetat, Chlorate oder auch Kaliumpermanganat als Oxidationsmittel dienen. Osmiumtetroxid wird mit Vorteil als Katalysator eingesetzt, kann jedoch auch in stöchiometrischer Menge verwendet, direkt als Oxidationsmittel dienen. Als Lösungsmittel werden vorteilhafterweise tertiär-Butanol oder Aceton verwendet. Wird $OsO_4$ in stöchiometrischer Menge eingesetzt, so hat sich insbesondere auch Pyridin als Lösungsmittel bewährt. Das Arbeiten im Zweiphasensystem unter Verwendung von Phasentransferkatalysatoren, wie z.B. Benzyltrimethylammoniumchlorid ist möglich (vgl. z.B. R. Criegée, M. Kropf, Methoden der organischen Chemie (Houben-Weyl), Bd. VI, Teil 1a, S. 592, Georg Thieme Verlag Stuttgart 1979). Das cis-1,2-Diol V wird anschliessend in das Monosulfonat der allgemeinen Formel VI übergeführt, wobei $R^7$ Niederalkyl oder Aryl bedeutet. Es finden die üblichen Methoden, z.B. unter Verwendung des entsprechenden Sulfochlorids $R^7$-$SO_2$-Cl in Pyridin, Anwendung.

Die in der Stufe VI$\longrightarrow$I (Verfahrensvariante c) erforderliche (isomerisierende) Ringkontraktion wird zweckmässigerweise durch Zugabe einer starken anorganischen Base, wie z.B. NaOH oder KOH in wässrig-alkoholischer Lösung bewerkstelligt. Die Reaktion läuft unter schonenden Temperaturbedingungen, vorzugsweise bei Raumtemperatur, ab und ist nach spätestens 24 Stunden beendet.

Je nach Art und Anzahl der Substituenten $R^1$ bis $R^6$ können die Verbindungen I bei der Herstellung als Diastereomeren- bzw. Strukturisomerengemische anfallen. Die

Trennung solcher Gemische in die einzelnen Komponenten kann z.B. mittels Gaschromatographie durchgeführt werden. Aus wirtschaftlichen Gründen werden daher vorzugsweise die Gemische verwendet.

Wie oben ausgeführt, ist eine Abtrennung der Nebenprodukte II' von II und III von I nicht nötig. Die Anwesenheit der Nebenprodukte zeigt keinen negativen Einfluss auf die sensorischen Qualitäten der neuen Verbindungen I.

Die Verbindungen der allgemeinen Formel I weisen besondere organoleptische Eigenschaften auf, auf Grund derer sie sich vorzüglich als Riech- und/oder Geschmackstoffe eignen.

Die Verbindungen der allgemeinen Formel I zeichnen sich insbesondere durch eine Kombination von fruchtigen, damasconartigen Moschusnoten aus, die bisher in der Palette des Parfumeurs fehlte. Einige Substanzen riechen zusätzlich in Richtung Ambrette-Samen bzw. weisen beerenartige Noten der Jonon-Richtung auf.

Die bevorzugten Verbindungen sind das 2,2,5,5-Tetramethylbicyclo[4.3.0]non-1(6)-en-8-yl-methylketon und das 2,2,3,5,5-Pentamethylbicyclo[4.3.0]non-1(6)-en-8-yl-methylketon.

Die erstgenannte bevorzugte Verbindung ist im übrigen auch gemäss Schema II zugänglich.

Reaktionsschema II

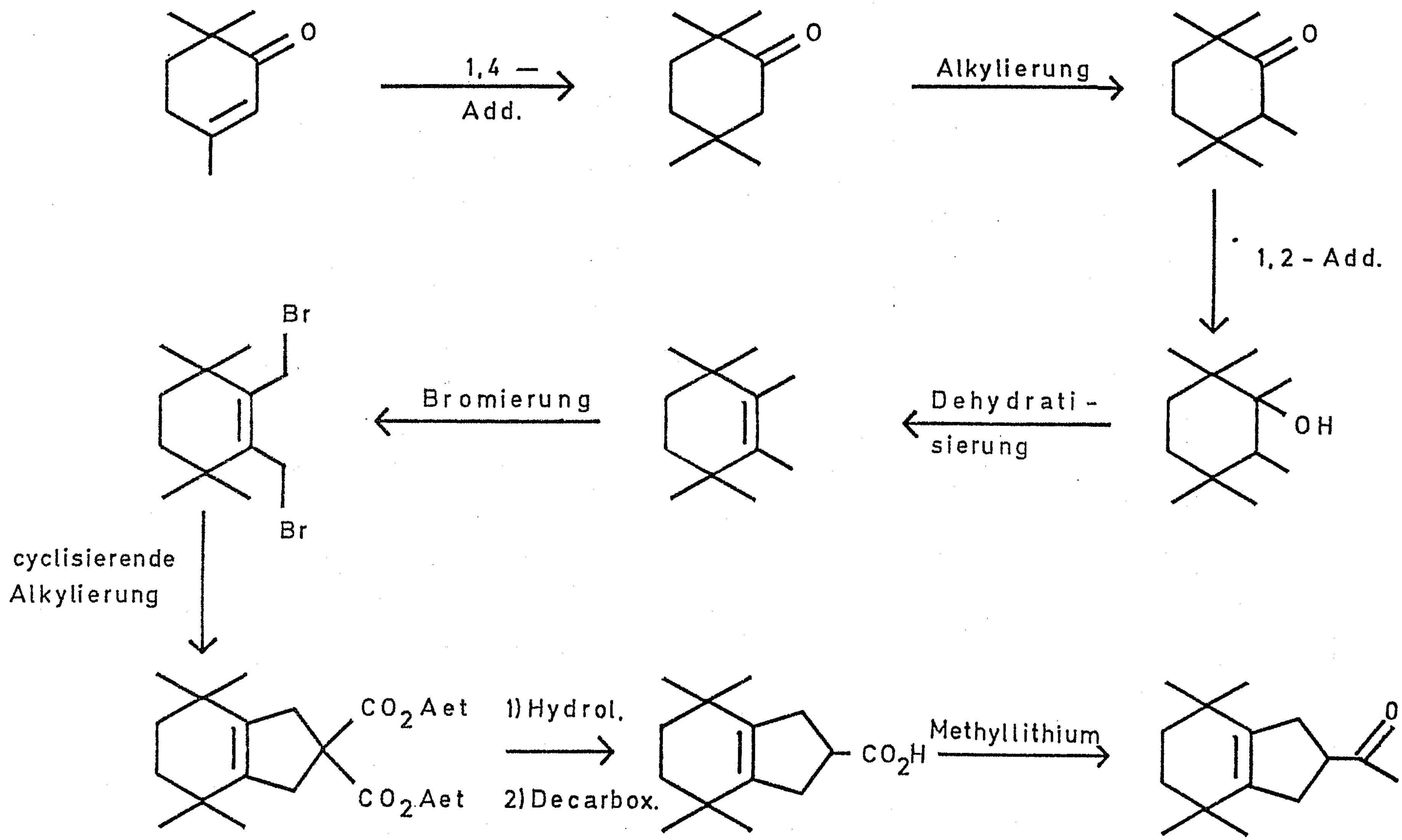

Es wird zunächst Methyllithium in Gegenwart eines $Cu^{1}$-Salzes an 3,6,6-Trimethylcyclohex-2-en-1-on addiert, anschliessend das gesättigte tetramethylsubstituierte Cyclohexanon mit MeJ in 2-Stellung methyliert und das Keton in bekannter Weise (1,2-Addition) zum tertiären Alkohol 1,2,3,3,6,6-Hexamethylcyclohexanol umgesetzt. Dieser Alkohol wird in Gegenwart von Mineralsäuren oder starken organischen Sulfonsäuren dehydratisiert, worauf sich das entstandene Hexamethylcyclohexen an den beiden an der C=C-Doppelbindung stehenden Methylgruppen bromieren lässt. Das so erhaltene Dibromid wird als Alkylierungsmittel zur cyclischen Alkylierung von Malonsäurediäthylester eingesetzt. Das dabei entstehende Reaktionsprodukt wird nach an sich bekannten Methoden verseift und decarboxyliert und liefert so die 2,2,5,5-Tetramethyl-bicyclo-[4.3.0]—non-1(6)-en-8-carbonsäure. In einem letzten Schritt wird als Reagens erneut Methyllithium eingesetzt, um aus der Carbonsäure in an sich ebenfalls bekannter Weise das entsprechende neue Methylketon zu erhalten.

Die Verbindungen der Formel I eignen sich aufgrund ihrer natürlichen Geruchsnoten insbesondere zur Modifizierung von bekannten, z.B.

α) blumigen Kompositionen, in denen z.B. die warmen Noten verstärkt zum Ausdruck kommen sollen (z.B. für Herren-Cologne),

β) des weiteren aber auch von fruchtigen, z.B. Typ Himbeere (Extrait-Typen, Kompositionen der femininen Richtung), von

γ) Tabak- und Holzkompositionen, (Extrait-Typen der masculinen Richtung) und schliesslich von

δ) Kompositionen mit grünen Noten, wo insbesondere eine erwünschte Abrundung und harmonisierende Effekte erzielt werden.

Als Riechstoffe eignen sich die Verbindungen I auf Grund ihrer oben beschriebenen, originellen Noten, insbesondere in Kombination mit einer Reihe von natürlichen und synthetischen Riechstoffen, wie z.B.

- Naturprodukten
  wie Angelikawurzöl, Galbanumöl, Vetiveröl, Patchouliöl, Sandelholzöl, Mandarinöl, Ylang Ylang Oel, Cedernöl, Fichtenöl, Lavendelöl, Bergamotteöl, Citronenöl, Orangenöl, Corianderöl, Eichenmoos, Castoreum, Ciste labdanum, Calmusöl, Geraniumöl, Jasmin absolue, Rosenöl, Cassis absolue, Narzissen absolue, Verveine absolue usw.

- Aldehyden
  wie $C_{10}$-, $C_{11}$-, $C_{14}$-, $C_{16}$-, $C_{18}$-Aldehyd, Hydroxycitronellal, Cyklamenaldehyd, Benzaldehyd, p.-tert.-Butyl-α-methylzimtaldehyd, Citral, Citronellal, 2,6-Dimethyl-5-hepten-1-al, Isovaleraldehyd, trans-2-Hexenal, Sorbinaldehyd, trans-2-Octenal, n-Octanal, n-Nonanal, trans-2-cis-6-Nonadienal, 2,4-Decadienal, Methylnonyl-acetaldehyd usw.

- Ketonen
  wie alpha-Jonon, beta-Jonon, Methyljonon, Allyljonon, Acetanisol, 4-(para-Hydroxyphenyl)-2-butanon, Campher, Menthon, Carvon, Pulegon usw.

- Acetalen und Ketalen
  wie Phenylacetaldehyd-dimethylacetal, Phenylacetaldehyd-glycerinacetal, 2-Methyl-1,3-dioxolan-2-äthylacetat, Capronaldehyd-dimethylacetal usw.

- Aethern
  wie Eugenolmethyläther, Methyl-1-methyl-cyclododecyläther, Anethol, Estragol usw.

- <u>Phenolkörpern</u>
  wie Vanillin, Eugenol, Isoeugenol, Creosol usw.

- <u>Alkoholen</u>
  wie Butanol, n-Hexanol, cis-3-Hexenol, trans-2,cis-6-Nonadienol, cis-6-Nonenol, Linalool, Geraniol, Nerol, Citronellol, Nerolidol, Farnesol, Benzylalkohol, Phenyläthylalkohol, Zimtalkohol usw.

- <u>Estern</u>
  wie Aethylformiat, Aethylacetat, Isoamylacetat, t-Butylcyclohexylacetat, Myraldylacetat ® (Givaudan), Benzylacetat, Styrallylacetat, Aethyl-α-methylphenyl-glycidat, Maltylisobutyrat, Dimethylbenzylcarbinylbutyrat, Linalylacetat, Isobutylacetat, n-Amylbutyrat, n-Amylvalerianat, Aethylpalmitat, Cinnamylformat, Terpenylacetat, Geranylacetat, Hexylsalicylat, Linalylanthranilat, Amylsalicylat, Methyldihydrojasmonat.

- <u>Lactone</u>
  wie γ-Undecalacton, γ-Decalacton, γ-Nonalacton, δ-Decalacton, δ-Octalacton, Cumarin usw.

- <u>Säuren</u>
  wie Milchsäure, Buttersäure, α-Methylbuttersäure, trans-2-Hexensäure, trans-2-Octensäure usw.

- <u>schwefelhaltigen Verbindungen</u>
  wie p-Menthan-8-thiol-3-on, Dimethylsulfid und anderen Sulfiden und Disulfiden usw.

- <u>stickstoffhaltigen Verbindungen</u>
  wie Methylanthranilat, Indol, Isobutylchinolin, verschiedenen Pyrazinen, 5-Methyl-heptan-3-on-oxim usw.

- Verschiedenen weiteren in der Parfümerie oft benutzten Komponenten
  wie Keton-Moschus, Musk 174 R (12-Oxahexadecanolid), Sandela (3-Isocamphyl-(5)-cyclohexanol).

Die Verbindungen der Formel I lassen sich in weiten Grenzen einsetzen, die beispielsweise von 0,1 (Detergentien) -50% (alkoholische Lösungen) in Kompositionen reichen können, ohne dass diese Werte jedoch Grenzwerte darstellen sollen, da der erfahrene Parfümeur auch mit noch geringeren Konzentrationen Effekte erzielen oder aber mit noch höheren Dosierungen neuartige Komplexe aufbauen kann. Die bevorzugten Konzentrationen bewegen sich zwischen 0,5 und 20%. Die mit I hergestellten Kompositionen lassen sich für alle Arten von parfümierten Verbrauchsgütern einsetzen (Eaux de Cologne, Eaux de Toilette, Extraits, Lotionen, Crèmes, Shampoos, Seifen, Salben, Puder, Desodorantien, Detergentien, Tabak, etc.).

Die Verbindungen I können demgemäss bei der Herstellung von Kompositionen und - wie obige Zusammenstellung zeigt - unter Verwendung einer breiten Palette bekannter Riechstoffe, verwendet werden. Bei der Herstellung solcher Kompositionen können die oben aufgeführten bekannten Riechstoffe nach (dem Parfümeur bekannter) Art und Weise verwendet werden, wie z.B. aus W.A. Poucher, Perfumes, Cosmetics and Soaps 2, 7. Auflage, Chapman und Hall, London, 1974 hervorgehend.

Die neuen Verbindungen der Formel I sind ebenfalls vorzüglich geeignet zur Verwendung in Fruchtaromen verschiedenster Art, insbesondere aber auch zur Aromatisierung von Tabak.

Als Geschmackstoffe können die Verbindungen I beispielsweise zur Erzeugung bzw. Verbesserung, Verstärkung, Steigerung oder Modifizierung von Fruchtaromen verschiedenster Art, z.B. Brombeer- oder Aprikosenaromen verwendet

werden. Als Anwendungsgebiet dieser Aromen kommen beispielsweise Nahrungsmittel (Joghurt, Süsswaren etc.), Genussmittel (Tee, Tabak etc.) und Getränke (Limonade etc.) in Frage.

Die ausgeprägten geschmacklichen Eigenschaften der Verbindungen I ermöglichen die Verwendung in geringen Konzentrationen. Eine geeignete Dosierung umfasst beispielsweise den Bereich von 0,01 - 100 ppm, vorzugsweise von 0,1 - 20 ppm im Fertigprodukt, d.h. dem aromatisierten Nahrungsmittel, Genussmittel oder Getränk.

Bei der Aromatisierung von beispielsweise Tabak kann die Dosierung jedoch auch höher liegen und einen grösseren Bereich umfassen, beispielsweise den Bereich von 1 bis 1000 ppm, vorzugsweise 50-500 ppm.

Die Verbindungen können auf übliche Weise mit den für Geschmackstoffkompositionen verwendeten Bestandteilen vermischt bzw. solchen Aromen zugesetzt werden. Unter den erfindungsgemäss verwendeten Aromen werden Geschmackstoffkompositionen verstanden, die sich auf an sich bekannte Art verdünnen bzw. in essbaren Materialien verteilen lassen. Sie enthalten beispielsweise etwa 0,1-10, insbesondere 0,5-3 Gew.%. Sie können nach an sich bekannten Methoden in die üblichen Gebrauchsformen, wie Lösungen, Pasten oder Pulver übergeführt werden. Die Produkte können sprühgetrocknet, vakuumgetrocknet oder lyophilisiert werden.

Die bei der Herstellung solcher Aromen zweckmässigerweise verwendeten bekannten Aromastoffe sind entweder in der obigen Zusammenstellung bereits enthalten oder können leicht der Literatur entnommen werden, wie z.B. J. Merory, Food Flavorings, Composition, Manufacture and Use, Second Edition, The Avi Publishing Company, Inc., Westport, Conn. 1968, oder G. Fenaroli, Fenaroli's Handbook of Flavor Ingredients, Second Edition, Volume 2, CRC-Press, Inc. Cleveland, Ohio, 1975.

Für die Herstellung solcher üblicher Gebrauchsformen kommen beispielsweise folgende Trägermaterialien, Verdickungsmittel, Geschmackstoffverbesserer, Gewürze und Hilfsingredientien, etc. in Frage:

Gummi arabicum, Tragant, Salze oder Brauereihefe, Alginate, Carrageen oder ähnliche Absorbentien; Indole, Maltol, Dienale, Gewürzoleoresine, Raucharomen; Gewürznelken, Diacetyl, Natriumcitrat; Mononatriumglutamat, Dinatriuminosin-5'-monophosphat (IMP), Dinatriumguanosin-5-phosphat (GMP); oder spezielle Aromastoffe, Wasser, Aethanol, Propylenglykol, Glycerin.

Beispiel 1

In einem Dreihals-Sulfierkolben mit Rührer und Tieftemperaturkühler (gefüllt mit $CO_2$/Isopropanol) werden 160 ml Methylamin vorgelegt und 40 g (0,2 M) 1,1,4,4,6-Pentamethyl-1,2,3,4-tetrahydro-naphthalin und 18 g (0,4 M) Aethanol zugegeben. Es wird auf -15°C abgekühlt, und es werden 2,7 g Lithium in kleinen Stücken zugegeben. Nach zehn Minuten gibt man noch einmal 9 g (0,2 M) Aethanol zu und nachträglich noch 1,4 g (0,2 M) Lithium in kleinen Stücken. Das ganze Lithium löst sich innerhalb fünfzehn Minuten auf. Dann wird das Methylamin abdestilliert und der Rückstand mit Hexan verdünnt. Die organische Phase wird mit Wasser neutral gewaschen, über Natriumsulfat getrocknet und eingedampft. Man erhält 40 g Rohprodukt. Dieses Produkt enthält (nach GC):

55% 2,2,5,5,8-Pentamethyl-bicyclo[4.4.0]deca-1(6),8-dien.
$^1$H-NMR ($CDCl_3$ 400 MHz): 0,99 (s, 3H); 1,01 (s, 3H); 1,5 (s, 4H); 1,68 (m, 3H); 2,54 (m, 2H); 2,65 (m, 2H); 5,42 (m, 1H);
MS: M+ 204, 189, 175, 161, 133, 119, 105, 93, 81.

7% 2,2,5,5,8-Pentamethyl-bicyclo[4.4.0]deca-1(10), 7,-dien.
$^1$H-NMR ($CDCl_3$ 400 MHz); 0,63 (s, 3H); 0,99 (s, 3H); 1,03 (s, 3H); 1,07 (s, 3H); 1,69 (s, 3H); 5,43 (m, 1H); 5,50 (m, 1H);
MS: 204, 189, 145, 135, 133, 119, 105, 91, 77, 69, 55, 41, 27.

30% 2,2,5,5,8-Pentamethyl-bicyclo[4.4.0]deca-1(6)-en.

6% Ausgangsmaterial.

253,3 g Kohlenwasserstoffgemisch, enthaltend rund 60% 2,2,5,5,8-Pentamethyl-bicyclo[4.4.0]deca-1(6),8-dien werden in 1050 ml tert.-Butanol gelöst und mit einer Lösung von 91,65 g Trimethyl-amin-N-oxid-dihydrat in 375 ml

Wasser versetzt. Dazu wird eine Lösung von 40 mg Osmiumtetroxid in 75 ml tert.-Butanol zugefügt und das Gemisch wird während 48 Stunden bei 95°C unter gutem Rühren bei Rückflusstemperatur gehalten. Die Lösung wird mit 2 l $CH_2Cl_2$, 1 l Wasser und 412,5 ml 2N Salzsäure und Eis extrahiert. Die organische Phase wird 2 mal mit je 1 l Wasser neutral gewaschen und die wässrigen Phasen mit $CH_2Cl_2$ extrahiert. Nach dem Trocknen über $MgSO_4$ und dem Einengen am Rotationsverdampfer verbleiben 269 g Rohkristalle. Diese werden aus 150 ml Hexan und 80 ml $CH_2Cl_2$ umkristallisiert. Man erhält 167,0 g kristallines 2,2,5,5,8-Pentamethyl-bicyclo[4.4.0]dec-1(6)-en-8,9-diol vom Schmelzpunkt 98-99,5°C.

$^1$H-NMR (400 MHz, $CDCl_3$): δ (ppm) 0,96 (s, 3H); 0,97 (s, 3H); 0,98 (s, 3H); 1,00 (s, 3H); 1,19 (s, 3H); 1,48 (s, 4H); 3,58 (m, 1H).

MS ($^m/e$): 238 ($M^+$), 220, 205, 177, 161, 149, 135, 121, 109, 105

150 g des Diols werden bei Raumtemperatur in 406,8 ml Pyridin unter Rühren vollständig gelöst. Zu dieser Lösung (gekühlt auf 0°C) tropft man während 30 Minuten 82,9 g Methansulfochlorid zu und rührt bei 20°C während 3 1/2 Stunden. Die Suspension wird auf 1 l $CH_2Cl_2$ gegossen und mit 2,77 l 2 N Salzsäure und mit Kochsalzlösung gewaschen; die wässrigen Phasen extrahiert man noch 2 mal mit je 500 ml $CH_2Cl_2$. Die vereinigten organischen Phasen werden über Magnesiumsulfat getrocknet und am Rotationsverdampfer eingeengt. Nach dem Trocknen am HV bei Raumtemperatur verbleiben 198,5 g kristallines Monomesylat des Diols, das, aus Dichlormethan-Hexan umkristallisiert, bei 100-104°C unter Zersetzung schmilzt.

198,5 g rohes Monomesylat werden bei Raumtemperatur in 1190 ml Methanol vollständig gelöst und mit 1190 ml 3 N KOH versetzt. Man rührt bei 20°C während 26 Stunden, nimmt in Hexan auf und wäscht mit Wasser; die wässrigen Phasen

0116277

werden 2 mal mit Hexan ausgezogen. Die vereinigten organischen Phasen werden über Magnesiumsulfat getrocknet und am Rotationsverdampfer eingeengt. Es verbleiben 134,3 g Oel, das über eine 15 cm Widmerkolonne destilliert wird. Die Fraktion mit Siedepunkt 70-76°C/0,03 Torr und $n_D^{20}$ 1,4868 (106,4 g) stellt reines 2,2,5,5-Tetramethyl-bicyclo-[4.3.0]-non-1(6)-en-8-yl-methylketon dar.

## Beispiel 2

200 g Kohlenwasserstoffgemisch, enthaltend 60%-iges 2,2,5,5,8-Pentamethyl-bicyclo[4.4.0]deca-1(6),8-dien werden in 2 l Methanol gelöst und unter Rühren werden 280 g (0,71 M) Thallium (III)nitrat zugegeben. Nach 48 Minuten Rühren bei Raumtemperatur wird eine Probe entnommen (GC). Die Reaktion ist noch nicht beendet. Nach 57 Minuten Rühren werden noch 50 g (0,12 M) Thallium(III) nitrat zugegeben. Nach zwei Stunden Rühren wird das Thallium(I)nitrat abfiltriert (184 g, 0,69 M). Die Lösung wird vorsichtig unter Rühren auf 160 g Natriumbicarbonat gegossen. Es wird während 30 Minuten bei Raumtemperatur gerührt. Das Methanol wird in Gegenwart von ein wenig Natriumbicarbonat am Rotationsverdampfer abdestilliert. Der Rückstand wird mit wenig Aether gewaschen. Das Rohprodukt (208 g) wird am Hochvakuum destilliert. Nach zweifacher Destillation (Widmer-Kolonne, Füllkörperkolonne 60 cm) erhält man 56 g Produkt.

Gaschromatographische Trennung und Analyse:

72% 2,2,5,5-Tetramethyl-bicyclo[4.3.0]non-1(6)-en-8-yl-methylketon.
$^1$H-NMR ($CDCl_3$ 400 MHz): 0,96 (s, 3H); 0,975 (s, 3H): 1,44 (s, 3H); 2,15 (s, 3H); 2,52 (d, J= 8 Hz, 4H); 3,09 (m, J= 8 Hz, $H_3$, 1H);
IR: 1710 $cm^{-1}$
MS: 220 $M^+$, 205, 177, 161, 145, 133, 119, 105, 91, 79, 55, 43.

Geruch: Ambrette-moschus, fruchtig, Damascon.

9% 1-(2,2,5,5-Tetramethyl-bicyclo[4.3.0]non-1(6)-en-7-yl)-1,1-dimethoxyäthan
$^1$H-NMR ($CDCl_3$ 400 MHz): 0,95 (s, 9H); 1 (s, 3H); 1,08 (s, 3H); 3,29 (s, 3H); 3,34 (q, J= 8, J = 6 Hz, 1H); 3,43 (s, 3H);
MS: 266, 234, 219, 203, 187, 175, 163, 147, 133, 119, 102, 91, 59.

## Beispiel 3

In einen 3-Hals-Sulfierkolben mit Rührer, Tieftemperaturkühler (gefüllt mit $CO_2$/Isopropanol) werden 600 ml Methylamin vorgelegt. Es werden nun 100 g 1,1,3,4,4,6-Hexamethyl-1,2,3,4-tetrahydro-naphthalin und 42,6 g Aethanol (0,92 M) zugegeben. Dann wird auf -15°C abgekühlt und 6,5 g (0,93 M) Lithium in kleinen Stücken innerhalb einer Stunde und fünfzehn Minuten zugegeben. Solange das Lithium nicht vollständig ausreagiert hat, wird auf -15°C gekühlt, dann wird über Nacht bei Rückflusstemperatur gehalten. Nach 19 1/2 Stunden sowie nach 25 1/2 Stunden werden jeweils nochmals je 3,25 g (0,46 M) Lithium innerhalb 30 Minuten bei -15°C zugegeben. Nach 29 Stunden wird das Methylamin abdestilliert. Der Rückstand wird mit Hexan verdünnt, viermal mit Wasser, einmal mit 5% Schwefelsäure und noch einmal mit Wasser (neutral) gewaschen, über Natriumsulfat getrocknet und eingedampft. Man erhält 82 g Rohprodukt. Dieses Produkt enthält (nach GC):

47% 2,2,4,5,5,8-Hexamethyl-bicyclo[4.4.0]deca-1(6),8-dien.
$^1$H-NMR ($CDCl_3$ 360 MHz): 0,83 (s, 3H); 0,88 (d, J = 7 Hz, 3H); 0,96 (s, 3H); 1 (s, 3H); 1,02 (s, 3H): 1,68 (s, 3H); 5,41 (m, 1H);
MS: 218, 203, 175, 159, 147, 133, 119, 105, 91, 83

29% 2,2,4,5,5,8-Hexamethyl-bicyclo[4.4.0]dec-1(6)-en.

In einem Dreihals-Sulfierkolben mit Rührer, Rückflusskühler, Thermometer, Schutzgaszuleitung mit Blasenzähler werden 154 rohes Dien (51% 0,357 M) in 660 ml t-Butanol gelöst, mit einer Lösung von 57 gr Trimethylamin-N-oxid in 232 ml Wasser versetzt. Dazu werden 150 ml einer 0,05%igen Lösung von Osmiumtetroxid in t-Butanol zugefügt. Die Emulsion wird während 48 Stunden unter gutem Rühren bei Rückflusstemperatur gehalten. Die abgekühlte Lösung wird mit 300 ml Aether verdünnt und mit gesättigter Kochsalzlösung (300 ml), 2 N HCl (325 ml) und Eis gewaschen. Die organische Phase wird zweimal mit gesättigter Kochsalzlösung gewaschen. Die wässrigen Phasen werden zweimal mit Aether extrahiert. Nach dem Trocknen der vereinigten organischen Phasen über $MgSO_4$ und dem Einengen im Rotationsverdampfer verbleiben 130 g Rohprodukt. Durch Umkristallisation in Hexan erhält man 85 g (94%) 2,2,4,5,5,8-Hexamethyl-bicyclo[4.4.0]dec-1(6)-en-8,9-diol in Form zweier Isomere.

$^1$H-NMR: ($CDCl_3$ 400 MHz): 0,79 (s, 6H); 0,87 (d, J= 7 Hz, 3H); 0,872 (d, J= 7 Hz, 3H); 0,92 (s, 3H); 0,98 (s, 3H); 0,97 (s, 3H); 0,972 (s, 3H); 1,02 (s, 6H); 1,1 (s, 3H); 1,29 (s, 3H);

MS: 252 $M^+$, 234, 219, 205, 191, 175, 161, 149, 135, 107, 91, 43

85 g (0,337 Mol) 2,2,4,5,5,8-Hexamethyl-bicyclo[4.4.0]-dec-1(6)-en-8,9-diol werden bei Raumtemperatur in 218 ml Pyridin gelöst. Es wird nun auf 0°C abgekühlt und 30,2 ml Methansulfochlorid werden während 20 Minuten langsam zugetropft. Man rührt das Gemisch bei Raumtemperatur 24 Stunden weiter. Man verdünnt hierauf mit Aether und wäscht mit 1,480 l 2 N Salzsäure und 400 ml gesättigter Kochsalzlösung. Die wässrige Phase wird mit Aether extrahiert und die vereinigten organischen Phasen werden mit verdünnter Natriumbicarbonatlösung neutralgewaschen, über Natriumsulfat getrocknet und eingedampft.

Spektrale Daten des Mesylats (2 Isomere):

$^1$H-NMR (CDCl$_3$ 400 MHz): 0,795 (s, 3H); 0,807 (s, 3H); 0,875 (d, J = 7 Hz, 3H) 0,877 (d, J = 7 Hz, 3H); 1,917 (s, 3H); 1,93 (s, 3H); 1,97 (s, 3H); 1,98 (s, 3H); 1,02 (s, 3H); 1,03 (s, 3H); 1,17 (s, 3H); 1,35 (s, 3H); 3,07 (s, 6H); 4,68 (m, 2H);

MS: 330, 312, 297, 234, 219, 201, 191, 175, 149, 135, 119, 105, 95, 83

100 g rohes Mesylat werden bei Raumtemperatur in 575 ml Methanol gelöst und diese Lösung mit 575 ml 3 N Kaliumhydroxid während 21 Stunden bei Raumtemperatur reagieren gelassen. Es wird hierauf mit Aether verdünnt und mit einer gesättigten Kochsalzlösung gewaschen. Die wässrige Phase wird mit Aether extrahiert. Die vereinigten organischen Phasen werden neutralisiert und über $MgSO_4$ getrocknet. Man erhält 73 g Rohprodukt. Durch Filtration mit Hexan über 700 g Kieselgel gewinnt man zunächst 11 g Hexamethyltetralin. Elution mit 20% Aether/Hexan ergibt 51 g 2,2,3,5,5-Pentamethyl-bicyclo[4.3.0]non-1(6)-en-8-yl-methylketon (2 Isomere).

$^1$H-NMR: (CDCl$_3$, 400 mHz): 1,78 (s, 3H); 1,79 (s, 3H); 1,86 (d, J = 7 Hz, 3H); 1,87 (d, J = 7 Hz, 3H); 1,95, 1,96, 1,965, 1,98 (4 s, 18H); 2,15 (s, 3H); 2,157 (s, 3H); 2,52 (m, 8H); 3,105 (m, 2H);

MS: 234 M$^+$, 219, 191, 175, 161, 149, 135, 119, 107, 91, 83, 69, 55, 43

IR: (flüssig) 1710 cm$^{-1}$

Geruch: nach Ambrette-Samen, Moschus.

## Beispiel 4

13 g Kohlenwasserstoffgemisch, enthaltend 28% 2,2,4,5,5,8-Hexamethyl-bicyclo[4.4.0]deca-1(6),8-dien werden in 80 ml Methanol gelöst, und es werden unter Rühren 6,5 g (0,01669 M) Thallium-III-nitrat zugegeben. Nach 30 Minuten Rühren bei Raumtemperatur wird das Thallium-(I)-nitrat

abfiltriert und die Lösung in Gegenwart von überschüssigem Natriumbicarbonat eingedampft. Das Rohprodukt wird über 300 g Kieselgel chromatographiert; mit 3% Aether in Hexan werden 900 mg 2,2,3,5,5-Pentamethyl-bicyclo[4.3.0]-non-1(6)-en-8-yl-methylketon und 670 mg 1-(2,2,4,5,5-Pentamethyl-bicyclo[4.3.0]-non-1(6)-en-7-yl)-1,1-dimethoxyäthan gewonnen.

Spektrale Daten des Ketals:

$^{1}$H-NMR: ($CDCl_3$, 400 MHz): 0,79 (s, 3H); 0,86 (d, J = 7 Hz, 3H); 0,955 (s, 3H); 0,962 (s, 3H); 0,995 (s, 3H); 1,155 (s, 3H); 3,21 (s, 3H); 3,25 (t, J = 5 Hz, 1H); 3,38 (s, 3H);

MS: 280 $M^+$, 248, 233, 217, 201, 189, 175, 163, 149, 133, 119, 102, 91, 73, 59

## Beispiel 5

In einem Dreihals-Sulfierkolben mit Rührer, Tieftemperaturkühler (gefüllt mit $CO_2$/Isopropanol) werden 300 ml Aethylamin vorgelegt, sodann 30 g (0,66 M) Aethanol und 48 g (0,22 M) 1,1,4,4,6,7-Hexamethyl-1,2,3,4-tetrahydronaphthalin zugegeben. Innerhalb einer Stunde werden 4,5 g (0,66 M) Lithium zugegeben. Man lässt unter Rückfluss reagieren. Nach drei Stunden Rühren gibt man noch einmal dieselbe Menge Lithium und Aethanol dazu und rührt über Nacht bei Raumtemperatur. Hierauf wird das Aethylamin abdestilliert und der Rückstand mit Hexan verdünnt. Die organische Phase wird mit Wasser neutral gewaschen, über Natriumsulfat getrocknet und eingedampft. Man erhält 43,9 g Rohprodukt. Dieses Produkt enthält (nach GC): 7% Ausgangsmaterial, 13% eines nicht isolierten Materials (wahrscheinlich Monoen) und 79% 2,2,5,5,8,9-Hexamethyl-bicyclo-[4.4.0]deca-1(6),8-dien.

$^{1}$H-NMR: ($CDCl_3$, 400 MHz): 1 (s, 12 H); 1,5 (s, 4H); 1,64 (s, 6H); 2,57 (s, 4H);

MS: 218 $M^+$, 203, 147, 133, 119, 91, 77, 69, 56, 41

Die Verbindung wird gaschromatographisch gereinigt.

In einem Sulfierkolben mit Rührer werden 26 g 2,2,5,5,8,9-Hexamethyl-bicyclo[4.4.0]deca-1(6),8-dien und 3 g Natriumacetat in 200 ml Methylenchlorid vorgelegt, das Ganze auf -20°C abgekühlt und 19 g 40%-ige Peressigsäure unter Rühren innerhalb 5 Minuten zugetropft. Man lässt 45 Minuten bei -10°C weiterrühren. Dann wird auf Wasser gegossen, mit gesättigter Natriumpyrosulfitlösung und Wasser gewaschen und eingedampft. Das Rohprodukt (26 g) enthält (nach GC) 70% 8,9-Epoxy-2,2,5,5,8,9-hexamethyl-bicyclo[4.4.0]-non-1(6)-en.

$^1$H-NMR: ($CHCl_3$, 400 MHz): 0,94 (s, 6H); 0,98 (s, 6H); 1,34 (s, 6H); 1,44 (m, 4H); 2,21 (d, J = 16 Hz, 2H); 2,46 (d, J = 16 Hz, 2H);

MS: 234, 219, 201, 191, 177, 161, 135, 119, 107, 91, 77, 55, 43.

Zu einer Lösung von 26 g des obigen rohen 8,9-Epoxy-2,2,5,5,8,9-hexamethyl-bicyclo[4.4.0]-non-1(6)-ens werden 7,5 g Bortrifluoridätherat unter Rühren zugegeben. Die Temperatur steigt auf 50°C. Man kühlt mit Eis. Nach 30 Minuten Rühren wird auf Wasser gegossen, mit gesättigter Natriumbicarbonatlösung gewaschen, getrocknet und eingedampft. Das Rohprodukt (27 g) wird über 200 g Kieselgel chromatographiert. Mittels Hexan werden 16 g 1,1,4,4,6,7-Hexamethyl-1,2,3,4-tetrahydro-naphthalin eluiert, mit 10% Aether in Hexan 6,5 g 2,2,5,5,8-Pentamethyl-bicyclo[4.3.0]non-1(6)-en-8-yl-methylketon.

$^1$H-NMR: ($CDCl_3$, 400 MHz): 0,94 (s, 6H); 0,96 (s, 6H); 1,2 (s, 3H); 1,45 (s, 4H); 2,08 (d, J = 13 Hz, 2H); 2,7 (d, J = 13 Hz, 2H); 2,13 (s, 3H);

MS: 234 $M^+$, 219, 191, 175, 161, 145, 135, 121, 105, 91, 83, 77, 69, 59, 55,

Beispiel 6

Zu einer Suspension von 20,8 g Aluminiumchlorid in 333 g Toluol wird eine Lösung von 240 g 3,6-Dichlor-3,6-dimethyl-heptan in 340 g Toluol innerhalb einer Stunde zugetropft. Man lässt 50 Minuten reagieren, gibt 15 g Aluminiumchlorid dazu und lässt nochmals 10 Minuten reagieren. Man giesst auf Eis und extrahiert mit Hexan. Die organische Phase wird mit Wasser gewaschen, über Natriumsulfat getrocknet und eingedampft. Man erhält 274 g Rohprodukt.

Spektrale Daten des Gemisches von 1,1,4,6-Tetramethyl-4-äthyl-1,2,3,4-tetrahydro-naphthalin und 1,4,4,6-Tetramethyl-1-äthyl-1,2,3,4-tetrahydro-naphthalin

$^1$H-NMR: ($CDCl_3$, 400 MHz): 0,76 (t, J = 7 Hz, 3H); 0,77 (t, J = 7 Hz, 3H); 1,207 (s, 3H); 1,22 (s, 3H); 1,23 (s, 3H); 1,24 (s, 3H); 1,27 (s, 3H); 1,28 (s, 3H); 2,29 (s, 3H); 2,30 (s, 3H); 6,94 (d breit, J = 8 Hz, 1H); 7,02 (s breit 1H); 7,105 (s, breit, 1H); 7,11 (d, J = 8 Hz, 1H); 7,2 (d, J = 8 Hz, 1H);

MS: 216, 201, 187, 173, 145, 131, 115, 105,

In einem 3-Hals-Sulfierkolben, versehen mit Rührer und Tieftemperaturkühler (gefüllt mit $CO_2$/Isopropanol) werden 700 ml Methylamin vorgelegt und 237 g eines Gemisches von 1,1,4,6-Tetramethyl-4-äthyl-1,2,3,4-tetrahydro-naphthalin und 1,4,4,6-Tetramethyl-1-äthyl-1,2,3,4-tetrahydro-naphthalin zugegeben. Es wird auf -15°C abgekühlt und mit zwei Portionen Aethanol (93 g, 47 g) und Lithium (15,2 g, 7,6 g) umgesetzt. Man hält 12 Stunden bei Rückflusstemperatur und gibt noch einmal 47 g Aethanol und 7,6 g Lithium zu. Hierauf wird das Methylamin abdestilliert. Der Rückstand wird mit Hexan verdünnt und mit Wasser gewaschen, getrocknet und eingedampft. Man erhält 233 g Rohprodukt. Dieses Produkt enthält nach GC (Flächenprozente):

40% eines Gemisches von 2,5,5,8-Tetramethyl-2-äthyl-bicyclo[4.4.0]-dec-1(6),8-dien und 2,2,5,8-Tetramethyl-5-äthyl-bicyclo[4.4.0]-dec-1(6),8-dien.

$^1$H-NMR: ($CDCl_3$, 400 MHz): 0,737 (3H, t, J = 7 Hz); 0,747 (3H, t, J = 7 Hz); 0,955 (s, 3H); 0,975 (s, 3H); 0,982 (s, 3H); 0,990 (s, 3H); 1,002 (s, 3H); 1,007 (s, 3H); 1,68 (s breit, 3H); 1,68 (s, 3H, breit); 5,42 (m, 1H); 5,42 (m, 1H);

MS: 218 $M^+$, 203, 189, 175, 173, 159, 147, 133, 119, 105, 91, 77, 69

45% eines Gemisches von cis- und trans-2,5,5,8-Tetramethyl-2-äthyl-bicyclo[4.4.0]-dec-1(6)-en und 2,2,5,8-Tetramethyl-5-äthyl-bicyclo[4.4.0]-dec-1(6)-en

$^1$H-NMR: ($CDCl_3$, 400 MHz): 0,892 (s); 0,905 (s); 0,925 (s); 0,945 (s); 0,950 (s); 0,950 (s); 0,965 (s);

MS: 220 $M^+$, 205, 191, 177, 149, 135, 121, 109, 105, 95.

10 g Kohlenwasserstoffgemisch, enthaltend 37% 2,5,5,8-Tetramethyl-2-äthyl-bicyclo[4.4.0]-dec-1(6),8-dien und 2,2,5,8-Tetramethyl-5-äthyl-bicyclo[4.4.0]-deca-1(6),8-dien werden in 200 ml Methanol gelöst und es werden unter Rühren 6,62 g $Tl(ONO_2)_3$ zugegeben. Nach 1,5 Stunden Rühren wird filtriert und in Gegenwart eines Ueberschusses von Natriumbicarbonat gerührt und eingedampft. Die Reinigung des Produktes erfolgt chromatographisch.

Spektrale Daten des Gemisches der cis- und trans-Isomeren von 2,5,5-Trimethyl-2-äthyl-bicyclo[4.3.0]-non-1,6-en-8-yl-methylketon.

$^1$H-NMR: ($CDCl_3$, 400 MHz): 0,76 (t, J = 7 Hz, 3H); 0,76 (t, J = 7 Hz, 3H); 0,917 (s, 3H); 0,927 (s, 3H); 0,955 (s, 3H); 0,965 (s, 3H); 0,970 (s, 3H); 0,977 (s, 3H); 2,154 (s, 3H); 2,155 (s, 3H); 3,080

(m, 1H); 3,085 (m, 1H);

IR: (flüssig): 1710 $cm^{-1}$

MS: 234 $M^+$, 219, 205, 191, 175, 161, 147, 131, 119, 105, 91, 77, 69, 43.

Geruch: Fruchtig, Moschus, schwach holzig.

## Beispiel 7

In einem Dreihals-Sulfierkolben mit Rührer werden 302 g konzentrierte Schwefelsäure vorgelegt, es wird auf -10°C abgekühlt und eine Lösung von 99 g 5-Methyl-hex-1-en-5-ol in 377 g Toluol wird langsam innerhalb von 1 1/4 Stunden zugetropft. Es wird noch 2 1/2 Stunden bei derselben Temperatur gerührt, dann mit Hexan verdünnt und auf Eis gegossen. Die wässrige Phase wird zweimal mit Hexan extrahiert. Die vereinigten organischen Phasen werden mit einer gesättigten Natriumcarbonatlösung gewaschen und über Natriumsulfat getrocknet. Das Rohprodukt (126 g) wird destilliert. Auf diese Weise erhält man 73,8 g 80%-iges 1,1,4,6-Tetramethyl-1,2,3,4-tetrahydro-naphthalin vom Siedepunkt 110°C/4 x $10^{-2}$ mm Hg.

$^1$H-NMR: ($CDCl_3$, 400 MHz): 1,24 (s, 3H); 1,275 (d, J = 7 Hz, 3H); 1,28 (s, 3H); 2,29 (s, 3H); 2,85 (m, 1H); 6,96 (d, J = 8 Hz, breit, 1H); 6,98 (s, breit, 1H); 7,21 (d, J = 8 Hz, 1H);

MS: 128 $M^+$, 173, 137, 143, 131, 115, 105, 91, 77, 65, 51, 41.

In einem Dreihals-Sulfierkolben mit Rührer, Tieftemperaturkühler (gefüllt mit $CO_2$/Isopropanol) werden 225 ml Aethylamin vorgelegt, dann 18,4 g (0,4 M) Aethanol und 38 g 1,1,4,6-Tetramethyl-1,2,3,4-tetrahydro-naphthalin (80%) zugegeben. Innerhalb zehn Minuten werden 2,8 g (0,4 M) Lithium zugegeben. Man lässt nun 1,5 Stunden bei Raumtemperatur rühren. Dann werden 10 g Aethanol (0,2 M) und 1,5 g Lithium hinzugefügt. Man lässt 2 1/2 Stunden reagieren. Hierauf wird das Aethylamin abdestilliert. Der

Rückstand wird mit Hexan verdünnt. Die organische Phase wird mit Wasser neutral gewaschen, über Natriumsulfat getrocknet und eingedampft. Man erhält 35 g Rohprodukt.

Dieses Produkt enthält (nach GC): 43% 2,2,5,8-Tetramethyl-bicyclo[4.4.0]deca-1(6),8-dien.

$^1$H-NMR: ($CDCl_3$, 400 MHz): 0,98 (s, 3H); 0,99 (s, 3H); 1,02 (d, J = 7 Hz, 3H); 1,67 (m, 3H); 5,43 (m, 1H);

MS: 190 $M^+$, 175, 147, 130, 119, 105, 81, 77, 65, 55, 41.

In einem Dreihalssulfierkolben mit Rührer, Rückflusskühler, Thermometer, Argonflow mit Blasenzähler werden 24 g rohes 2,2,5,8-Tetramethyl-bicyclo[4.4.0]deca-1(6),8-dien (43%) in 120 ml t-Butanol mit einer Lösung von 10,5 g Trimethylamin-N-oxid in 43 ml Wasser versetzt. Dazu fügt man 50 ml einer 0,05%igen Lösung von Osmiumtetroxid in t-Butanol. Die entstandene Emulsion wird während 96 Stunden unter gutem Rühren bei Rückflusstemperatur gehalten. Die abgekühlte Lösung wird mit Aether verdünnt und mit einer gesättigten Kochsalzlösung, mit 2 N HCl und mit Eiswasser gewaschen. Die wässrigen Phasen werden zweimal mit Aether extrahiert. Nach dem Trocknen der vereinigten organischen Phasen über $MgSO_4$ und dem Einengen im Rotationsverdampfer verbleiben 24 g Rohprodukt. Durch Umkristallisation in Hexan werden 6 g 2,2,5,9-Tetramethyl-bicyclo-[4.4.0]-dec-1(6)-en-8,9-diol erhalten. Schmelzpunkt 121-123°C.

$^1$H-NMR: ($CDCl_3$, 400 MHz): 0,975 (d, J = 7 Hz, 3H); 0,977 (s, 3H); 0,99 (s, 3H); 1,22 (s, 3H); 3,59 (q, J = 6 Hz, J = 13 Hz, 1H);

MS: 224 $M^+$, 206, 191, 177, 163, 150, 135, 121, 107, 95, 74, 55, 43.

5 g (0,0265 Mol) 2,2,5,9-Tetramethyl-bicyclo[4.4.0]-dec-1(6)-en-8,9-diol werden bei Raumtemperatur in 17 ml Pyridin gelöst. Es wird alsdann auf 0°C abgekühlt und es werden 2,36 ml Methansulfochlorid während 20 Minuten langsam zugetropft. Man rührt bei Raumtemperatur 24 Stunden. Man verdünnt mit Aether und wäscht mit 1,480 l 2 N Salzsäure und 400 ml gesättigter Kochsalzlösung. Die wässrige Phase wird mit Aether extrahiert. Die vereinigten organischen Phasen werden mit einer verdünnten Natriumbicarbonatlösung neutral gewaschen, über Natriumsulfat getrocknet und eingedampft. Man erhält 4,1 g rohes Monomesylat.

$^1$H-NMR: ($CDCl_3$, 400 MHz): 0,97 (d, J = 7 Hz, 3H); 0,985 (s, 3H); 1 (s, 3H); 1,29 (s, 3H); 3,07 (s, 3H); 4,68 (q, J = 6 Hz, J = 7 Hz, 1H);

MS: 287, 267, 206, 173, 163, 147, 131, 121, 107, 95, 91, 79, 69, 57, 41.

4 g rohes Mesylat werden bei Raumtemperatur in 25,1 ml Methanol gelöst und mit 25,1 ml 3 N Kaliumhydroxid während 12 Stunden bei Raumtemperatur gerührt. Es wird mit Aether verdünnt und mit einer gesättigten Kochsalzlösung gewaschen. Die wässrige Phase wird mit Aether extrahiert. Die vereinigten organischen Phasen werden neutralisiert und über $MgSO_4$ getrocknet. Man erhält 4 g Rohprodukt. Durch Filtration mit Hexan über Kieselgel erhält man zunächst 1 g 1,1,4,6-Tetramethyltetralin. Elution mit 20% Aether/Hexan ergibt 1,8 g 2,2,5-Trimethyl-bicyclo[4.3.0]-non-1(6)-en-8-yl-methylketon.

$^1$H-NMR: ($CDCl_3$, 400 MHz); 0,965 (s, 3H); 0,9725 (d, J = 7 Hz, 3H); 0,98 (s, 6H); 2,16 (s, 3H);

MS: 206 $M^+$, 191, 173, 163, 147, 133, 119, 107, 105, 91, 77, 69, 55, 43

Geruch: Damasconartig, jononartig: nach Beeren, Tabak.

Beispiel 8

In einem Dreihalssulfierkolben mit Rührer, Tieftemperaturkühler (gefüllt mit $CO_2$/Isopropanol) werden 250 ml Aethylamin vorgelegt. Es werden 2,36 g Methanol und 8 g 1,1,6-Trimethyl-4-isopropyl-1,2,3,4-tetrahydronaphthalin zugegeben. Innerhalb einer Minute addiert man 0,5 g (0,074 M) Lithium. Man lässt 12 Stunden unter Rückfluss reagieren. Hierauf gibt man noch einmal dieselbe Menge Lithium und Methanol zu und rührt 7 Stunden bei Raumtemperatur. Dann wird das Aethylamin abdestilliert und der Rückstand mit Hexan verdünnt. Die organische Phase wird mit Wasser neutral gewaschen, über Natriumsulfat getrocknet und eingedampft. Man erhält 6,9 g Rohprodukt. Dieses Produkt enthält (nach GC): 88% 2,2,8-Trimethyl-5-isopropyl-bicyclo[4.4.0]-nona-1(6),8-dien.

$^1$H-NMR: ($CDCl_3$, 400 MHz): 0,71 (d, J = 7 Hz, 3H); 0,945 (d, J = 7 Hz, 3H); 0,975 (s, 3H); 0,98 (s, 3H); 1,66 (s, breit 3H); 5,43 (m, 1H);

MS: 218 $M^+$, 175, 159, 145, 133, 119, 105, 91.

6,9 g 2,2,8-Trimethyl-5-isopropyl-bicyclo[4.4.0]-nona-1(6),8-dien werden in 70 g Methanol gelöst und unter Rühren werden 12 g Thallium-(III)-nitrat zugegeben. Nach 20 Minuten Rühren bei Raumtemperatur wird das gebildete Thallium(I)-nitrat abfiltriert und die Lösung in Gegenwart eines Ueberschusses an Natriumbicarbonat eingedampft. Das Rohprodukt wird über 100 g Kieselgel chromatographiert. Man erhält 2 g 2,2-Dimethyl-5-isopropyl-bicyclo[4.3.0]-non-1(6)-en-8-yl-methylketon.

$^1$H-NMR: ($CDCl_3$, 400 MHz): 0,712 (d, J = 7 Hz, 3H); 0,93 (d, J = 7H, 3H); 0,965 (s, 6H); 2,165 (s, 3H); 3,115 (m, 1H);

MS: 234 $M^+$, 219, 191, 173, 163, 147, 133, 119, 105, 91, 77, 69, 55, 43

IR: (flüssig) 1710 $cm^{-1}$

Geruch: Fruchtig, damasconartig.

Beispiel 9

7,3 g 4,5,6,7-Tetrahydro-4,4,7,7-tetramethyl-2-indancarbonsäure werden in 150 ml Tetrahydrofuran gelöst und unter Eiskühlung und kräftigem Rühren langsam mit 47,3 ml Methyllithium (1,6 molar in Aether) versetzt. Nach 3-stündigem Rühren bei Raumtemperatur giesst man auf Eis, extrahiert mit Aether, wäscht mit Wasser, trocknet über Natriumsulfat und dampft ein. Nach Destillation des Rohproduktes erhält man 5,6 g 2,2,5,5-Tetramethyl-bicyclo[4.3.0]non-1(6)-en-8-yl-methylketon als farblose Flüssigkeit, Siedepunkt 53-60°C/0,01 mm Hg.

Die als Ausgangsprodukt verwendete 4,5,6,7-Tetrahydro-4,4,7,7-tetramethyl-2-indancarbonsäure lässt sich wie folgt herstellen:

5,3 g Magnesiumspäne werden mit 100 ml Aether überschichtet und 30,8 g Methyljodid derart zugetropft, dass das Reaktionsgemisch leicht siedet. Nach Zugabe von 100 ml Aether hält man noch 3 Stunden bei Rückflusstemperatur, kühlt mittels Eisbad und versetzt mit 20,7 g Kupfer(I)jodid. Nach 15-minütigem Rühren bei 0°C tropft man eine Lösung von 10 g 3,6,6-Trimethyl-2-cyclohexenon in 40 ml Aether zu und rührt weitere 3 Stunden bei 0°C. Anschliessend giesst man die grüne Suspension auf Eis, säuert mit 2 N Salzsäure an und extrahiert mit Aether. Die unlöslichen Anteile der organischen Phase werden abfiltriert, das Filtrat mit verdünnter Natriumthiosulfatlösung gewaschen, getrocknet und eingedampft. Nach Destillation des Rohproduktes erhält man 8,5 g 2,2,5,5-Tetramethylcyclohexanon als farblose Flüssigkeit, Siedepunkt 60-67°C/13 mm Hg.

Zu einer Lösung von 21,6 g Diisopropylamin in 340 ml Aethylenglycoldimethyläther tropft man unter Eiskühlung 106,9 ml n-Butyllithium (2 molar in Hexan). Nach 30-minütigem Rühren bei 0°C tropft man eine Lösung von 23,2 g 2,2,5,5-Tetramethylcyclohexanon in 100 ml Aethylenglycoldimethyläther unter Eiskühlung zu. Man rührt noch 45 Minuten bei Raumtemperatur, kühlt erneut auf 0°C und gibt schnell 213,9 g Methyljodid hinzu. Man rührt 45 Minuten bei 50°C, kühlt ab, giesst das Reaktionsgemisch auf Eis und extrahiert mit Aether. Nach dem Waschen der organischen Phase mit 5% Salzsäure und verdünnter Natriumhydrogencarbonatlösung trocknet man über Natriumsulfat und dampft ein. Die Destillation des Rohproduktes an einer Drehbandkolonne ergibt 13,7 g 2,2,5,5,6-Pentamethylcyclohexanon als farblose Flüssigkeit, Siedepunkt 86-94°C/16 mm Hg.

26 g 2,2,5,5,6-Pentamethylcyclohexanon werden in 500 ml Aether gelöst und bei -20°C langsam mit 113,5 ml Methyllithium (1,5 molar in Aether) versetzt. Nach 3,5-stündigem Rühren bei 0°C giesst man auf Eis, säuert mit 1 N Salzsäure an, extrahiert mit Essigester, trocknet und dampft ein. Destillation des Rohproduktes ergibt 25,9 g 1-Hydroxy-1,2,2,5,5,6-hexamethylcyclohexan als farblose Flüssigkeit, Siedepunkt 87-92°C/ 10 mm Hg.

25,9 g 1-Hydroxy-1,2,2,5,5,6-hexamethylcyclohexan werden in 600 ml Benzol gelöst und nach Zugabe einer Spatelspitze p-Toluolsulfonsäure 3 Stunden am Wasserabscheider bei Siedetemperatur gehalten. Man kühlt ab, versetzt mit etwas Soda, rührt kurz, filtriert und destilliert das Benzol bei Normaldruck ab. Destillation des Rückstandes ergibt 21 g 1,2,3,3,6,6-Hexamethyl-1-cyclohexen als farblose Flüssigkeit, Siedepunkt 65-67°C/10 mm Hg.

21 g 1,2,3,3,6,6-Hexamethyl-1-cyclohexen werden in 600 ml Tetrachlorkohlenstoff gelöst. Nach Zugabe von 45 g N-Bromsuccinimid und einer Spatelspitze α,α'-Azoisobutyronitril hält man 3 Stunden bei Rückflusstemperatur, kühlt ab, filtriert das entstandene Succinimid ab und dampft ein. Nach Filtration des Rohproduktes über Kieselgel (Elutierungsmittel Hexan/Aether = 2:1) erhält man 38 g 1,2-Dibrom-

methyl-3,3,6,6-tetramethyl-1-cyclohexen als farbloses Oel, das im Tiefkühlschrank erstarrt.

38 g 1,2-Dibrommethyl-3,3,6,6-tetramethyl-1-cyclohexen und 18,7 g Malonsäurediaethylester werden in 150 ml Aethanol gelöst und auf 80°C erwärmt. Bei dieser Temperatur tropft man eine Lösung von 5,4 g Natrium in 250 ml Aethanol zu. Anschliessend hält man noch 6 Stunden bei Rückflusstemperatur. Nach dem Abkühlen giesst man auf Eis, säuert mit 1 N Salzsäure an, extrahiert mit Aether, wäscht mit Wasser und dampft ein. Dieses Rohprodukt wird an Kieselgel chromatographiert (Eluierungsmittel Hexan/Aether = 4:1). Man erhält 18,4 g 4,5,6,7-Tetrahydro-4,4,7,7-tetramethyl-2,2-indanbiscarbonsäure-äthylester als nicht ganz einheitliches farbloses Oel. Die weitere Reinigung erfolgt in der nächsten Stufe.

18,4 g 4,5,6,7-Tetrahydro-4,4,7,7-tetramethyl-2,2-indanbiscarbonsäure-äthylester werden in 500 ml Aethanol gelöst und mit einer Lösung von 64 g Kaliumhydroxid in einem Gemisch von 200 ml Aethanol und 50 ml Wasser versetzt. Nach 6-stündigem Erhitzen auf Rückflusstemperatur kühlt man ab, destilliert die Hauptmenge des Alkoholes am Rotationsverdampfer ab, giesst den Rückstand auf Eis, säuert mit 2 N Salzsäure an, extrahiert mehrfach mit Essigester, trocknet und dampft ein. Das so erhaltene Rohprodukt wird 5,5 Stunden auf 160°C erwärmt und anschliessend an Kieselgel chromatographiert (Eluierungsmittel Hexan/Aether = 2:1). Nach dem Umkristallisieren aus Pentan erhält man 6,5 g 4,5,6,7-Tetrahydro-4,4,7,7-tetramethyl-2-indancarbonsäure in farblosen Kristallen, Schmelzpunkt 125-129°C.

In den folgenden Formulierungsbeispielen steht jeweils:

"Verbindung 1" für 2,2,5,5-Tetramethylbicyclo-[4.3.0]non-1(6)-en-8-yl-methylketon.

"Verbindung 2" für 2,2,3,5,5-Pentamethylbicyclo-

[4.3.0]non-1(6)-en-8-yl-methylketon.

"Verbindung 3" für 2,2,5-Trimethylbicyclo[4.3.0]-non-1(6)-en-8-yl-methylketon.

"Verbindung 4" für 2,5,5-Trimethyl-2-äthylbicyclo-[4.3.0]non-1(6)-en-8-ylmethylketon.

"Verbindung 5" für 2,2,5,5,8-Pentamethylbicyclo-[4.3.0]non-1(6)-en-8-yl-methylketon.

## Beispiel 10

### A. Himbeeraroma

| | Gewichtsteile | |
|---|---|---|
| Palmitinsäure-äthylester | 0,05 | 0,05 |
| Geraniol | 0,2 | 0,2 |
| Methyljonon | 0,6 | 0,6 |
| Aethylvanillin | 1,0 | 1,0 |
| Valeriansäure-amylester | 1,0 | 1,0 |
| Essigsäure-benzylester | 2,0 | 2,0 |
| C 16-Aldehyd | 2,5 | 2,5 |
| Ameisensäure-äthylester | 4,0 | 4,0 |
| Essigsäure-amylester | 6,0 | 6,0 |
| Buttersäure-äthylester | 6,0 | 6,0 |
| Essigsäure-i-butylester | 23,0 | 23,0 |
| Essigsäure-äthylester | 33,5 | 33,5 |
| Propylenglykol | 920,15 | 905,15 |
| Verbindung 1 (10% in Aethanol) | -- | 15,0 |
| | 1'000,00 | 1'000,00 |

Durch den Zusatz von Verbindung 1 zu obiger Komposition wird die vorhandene Methyljonon-Note geruchlich und geschmacklich sehr vorteilhaft verändert, es tritt eine fruchtige Note in Erscheinung, die an vollreife Himbeeren erinnert.

B. Tabakaroma

| | Gewichtsteile | |
|---|---|---|
| Essigsäure-linalylester 10% in Aethanol | 0,3 | 0,3 |
| Zimtaldehyd 10% Alk. | 0,4 | 0,4 |
| Geraniol | 0,5 | 0,5 |
| Angelikawurzelöl | 0,5 | 0,5 |
| Buttersäure-amylester | 1,0 | 1,0 |
| Valeriansäure-amylester | 1,0 | 1,0 |
| Vanillin | 2,0 | 2,0 |
| C 18-Aldehyd | 2,0 | 2,0 |
| Petitgrainöl Paraguay | 2,0 | 2,0 |
| Benzaldehyd | 2,5 | 2,5 |
| Orangenöl 10-fach konzentriert | 5,0 | 5,0 |
| C 14-Aldehyd | 15,0 | 15,0 |
| Alkohol | 967,8 | 957,8 |
| Verbindung 1 | -- | 10,0 |
| | 1'000,0 | 1'000,0 |

Durch den Zusatz von Verbindung 1 zu obiger Komposition wird die in der Komposition vorhandene fruchtige Note deutlich verstärkt. Die leichte fettige Note wird vorteilhaft überdeckt, es tritt eine zusätzliche Note in Erscheinung, die an vollreife Aprikosen erinnert.

Bei der Einarbeitung der Komposition in Tabak ist die verstärkte fruchtige Note von Vorteil, zusätzlich wird die vorhandene Tabaknote in vorteilhafter Weise verstärkt.

C. Allgemein Blumige Parfümerie Base

| | Gewichtsteile |
|---|---|
| Terpineol | 260 |
| Hydroxycitronellal | 220 |
| Zimtalkohol-substitut | 120 |
| Phenyläthylalkohol | 100 |
| Cinnamylformiat | 20 |
| Linalool | 15 |
| Terpenylacetat | 10 |

| | |
|---|---|
| Keton-moschus | 10 |
| Geranylacetat | 10 |
| Jasmin synth. | 10 |
| Eugenol | 5 |
| Indol 10% in Dipropylenglykol (DPG) | 5 |
| C-10-Aldehyd 10% in DPG | 5 |
| p-Methylacetophenon | 5 |
| Undecalacton | 5 |
| | 500 |

Durch Zugabe von 200 Teilen der Verbindung 1 wird die Komposition viel weicher, sie erhält eine Note in Richtung Erdbeere; sie ist nun insbesondere zum Parfümieren von Kosmetika geeignet.

D. Parfümerie-Komplex Richtung Eau de Cologne

| | Gewichtsteile |
|---|---|
| Bergamotte-öl | 200 |
| Methyldihydrojasmonat | 200 |
| Sandelholzöl | 200 |

Gibt man zu diesem Komplex 50 Teile der Verbindung 1 so wird der dominierende Sandel-Charakter der Fondnote vorteilhaft eingekleidet, und zwar mittels sehr warmer, sehr kräftiger Moschus-Note, welche dem Komplex wesentlich mehr Volumen verleiht. Frisch getaucht verbindet sich nun der markante Citrus-Effekt sehr eindrucksvoll mit der Moschus-Note der neuen Substanz. Dieser einfache Komplex kann für Herren-Colognes sehr gut Anwendung finden.

E. Parfümerie-Komposition mit Rosen-Charakter

| | Gewichtsteile |
|---|---|
| Phenyläthylalkohol | 300 |
| Geraniol | 250 |
| Jasmin "lavage" (wässriges Destillat) | 200 |
| Citronellol extra | 100 |
| Keton-Moschus | 50 |
| α-Jonon | 30 |

| | |
|---|---|
| C-10-Aldehyd 10% in Propylenglykol | 5 |
| C-11-Aldehyd 10% in Propylenglykol | 5 |
| | 940 |

Durch Zusetzen von 60 Teilen der Verbindung 1 wirkt die Rosen-Base viel runder, weicher und erhält zudem einen fruchtigen Charakter.

F. Parfümerie Chypre

| | Gewichtsteile |
|---|---|
| 1-Methylcyclododecylmethyläther | 200 |
| Bergamotte-öl | 150 |
| Hydroxycitronellal | 100 |
| Fichten-öl Pumillon | 80 |
| Citronellol | 80 |
| Petitgrain-öl | 60 |
| Musc 174 ® Giv (12-Oxahexadecanolid) | 60 |
| Coriander-öl | 40 |
| Galbanum-öl | 40 |
| Zedernholz-öl | 40 |
| Patchouli-öl | 40 |
| Citronen-öl | 40 |
| Elemi-öl | 10 |
| Eichenmoos entfärbt | 20 |
| | 960 |

Durch Zugabe von 40 Teilen der Verbindung 1 wirkt diese Chypre-Komposition sowohl frisch getaucht als auch im Fond viel kräftiger. Die neue Verbindung zeigt hier auf anschauliche Weise den die Komponenten verbindenden Effekt.

G. Parfümerie-Base mit Fruchtcharakter

| | Gewichtsteile |
|---|---|
| Aethyl-methyl-phenyl-glycidat | 50 |
| Aethyl-acetyl-acetat | 15 |
| Dimethyl-benzyl-butyrat | 15 |
| Maltyl-isobutyrat | 10 |

| | |
|---|---|
| Benzylacetat | 10 |
| Aethyl-acetat | 5 |
| Citronenöl | 5 |
| Dipropylenglykol | 890 |
| | 970 |

Gibt man zu dieser allgemein fruchtigen Base 30 Teile der Verbindung 1, so wird diese sehr angenehm in Richtung exotische Frucht abgewandelt. Sie wirkt nun viel weicher und frischer. Ueberraschenderweise bewirkt die Verbindung auch eine Bereicherung der Kopfnoten.

H. Parfümerie-Base mit allgemein blumigem Charakter

| | Gewichtsteile |
|---|---|
| Terpineol | 260 |
| Hydroxycitronellal | 220 |
| Zimtalkohol-substitut | 100 |
| Cinnamylformiat | 20 |
| Linalool | 15 |
| Terpenylacetat | 10 |
| Ketonmoschus | 10 |
| Geranylacetat | 10 |
| Jasmin synthetisch | 10 |
| Eugenol | 5 |
| Indol 10% in DPG | 5 |
| C-10-Aldehyd 10% in DPG | 5 |
| p-Methylacetophenon | 5 |
| Undecalacton | 5 |
| DPG | 100 |
| | 900 |

Ein Zusatz von 100 Teilen der Verbindung 2 erbringt einen sehr angenehmen Moschus-Charakter, der diese vorher bloss allgemein blumige Base für die Parfümerie verwendbar macht. Mit 100 Teilen der Verbindung 3 wird andererseits ein schöner Veilcheneffekt erzielt.

J. Parfümerie-Base mit fruchtigem Charakter

| | Gewichtsteile |
|---|---|
| Aethyl-methyl-phenyl-glycidat | 50 |
| Aethyl-acetyl-acetat | 15 |
| Dimethyl-benzyl-butyrat | 15 |
| Maltylisobutyrat | 10 |
| Benzylacetat | 10 |
| Aethylacetat | 5 |
| Citronen-öl | 5 |
| Dipropylenglykol | 795 |
| | 900 |

Durch 100 Teile der Verbindung 4 wird aus der allgemein fruchtigen Komposition eine sehr süsse Apfelkomposition. Der Zusatz von 100 Teilen der Verbindung 2 andererseits erbringt einen Thibetolid-Effekt (Moschuseffekt), welcher den Eindruck einer Brombeere vermittelt.

K. Parfümerie-Base in Richtung Chypre

| | Gewichtsteile |
|---|---|
| 1-Methyl-cyclododecylmethyläther | 200 |
| Bergamotte-öl | 150 |
| Hydroxycitronellal | 100 |
| Fichtennadel-öl | 80 |
| Citronellol | 80 |
| Petitgrain-öl | 60 |
| Coriander-öl | 40 |
| Galbanum-öl | 40 |
| Zedernholz-öl | 40 |
| Patchouli-öl | 40 |
| Citronen-öl | 40 |
| Elemi-öl | 10 |
| Eichenmoos entfärbt | 20 |
| Dipropylenglykol | 60 |
| | 960 |

Mittels 40 Teilen der Verbindung 4 erzielt man in der ursprünglich unspezifischen Chypre-Base einen interessanten

fruchtigen Effekt. 40 Teile der Verbindung 3 vermitteln einen schönen Veilcheneffekt.

L. Parfümerie-Base in Richtung Rose

| | Gewichtsteile |
|---|---|
| Phenyläthylalkohol | 300 |
| Geraniol | 250 |
| Jasmin "lavage" (wässriges Destillat) | 200 |
| Citronellol extra | 100 |
| α-Jonon | 40 |
| C-10-Aldehyd 10% in Dipropylenglykol | 5 |
| C-11-Aldehyd 10% in Dipropylenglykol | 5 |
| | 900 |

Der Zusatz von 100 Teilen der Verbindung 2 bringt einen Charakter, der sonst nur mit dem viel teureren Thibetolid erzielt werden kann. Der Zusatz von 100 Teilen der Verbindung 3 bringt einen sehr schönen Veilcheneffekt.

M. Parfümerie-Base in Richtung Tabak

| | Gewichtsteile |
|---|---|
| α-tert.-Butylcyclohexylacetat | 400 |
| Jasminöl synthetisch | 300 |
| Ketonmoschus | 40 |
| Sandela ® (3-Isocamphyl-(5)-cyclohexanol) | 40 |
| Styrallylacetat | 30 |
| Cumarin | 20 |
| Isobutylchinolin 10% DPG | 10 |
| Lavendelöl | 10 |
| Vetiveröl | 10 |
| Galbanumöl | 10 |
| Vassuraöl | 10 |
| Dipropylenglykol | 40 |
| | 920 |

Durch Zusetzen von 80 Teilen der Verbindung 5 bewirkt man in der Tabakbase einen schönen krautigen Nebeneffekt. Mit 80 Teilen der Verbindung 3 erzielt man eine Veilchen-Note

in dieser Base. Eine Moschus-Note bringt ein Zusatz von 80 Teilen der Verbindung 2.

N. Parfümerie-Base in Richtung Tulpe

| | Gewichtsteile |
|---|---|
| Phenyläthylalkohol | 100 |
| Myraldehylacetat® [4-(4-Methyl-3-pentenyl)-3-cyclohexen-1-yl]methylacetat | 100 |
| Methyldihydrojasmonat | 100 |
| Acetal CD (Glycerinacetal von Phenylacetaldehyd) | 100 |
| Hydroxycitronellal | 160 |
| Farnesol | 40 |
| Hexylsalicylat | 30 |
| Terpineol | 30 |
| Cyklamenaldehyd | 20 |
| Linalool | 20 |
| Linalylanthranilat | 10 |
| Amylsalicylat | 10 |
| C-11-Aldehyd 10% DPG | 10 |
| Benzylacetat | 8 |
| Hexenylbenzoat | 8 |
| Hexenylacetat 10% DPG | 8 |
| p-Cresyl-isobutyrat 10% DPG | 6 |
| Indol 10% DPG | 6 |
| Syringaaldehyd | 4 |
| Dimethylacetal-hydratropaldehyd 10% DPG | 30 |
| DPG | 100 |
| | 900 |

Aus dieser Phantasiekomposition (Tulpe) lässt sich mit einem Zusatz von 100 Teilen der Verbindung 4 eine sehr schöne fruchtige Heckenrose erzielen, was einer eindeutigen Veredelung entspricht. Mit dem gleichen Zusatz von Verbindung 2 bringt man sowohl in der Fond- als auch in der Kopfnote eine schöne Moschus-Note in Richtung Thibetolid. Durch den Zusatz von 100 Teilen der Verbindung 3 wird aus der Tulpe ein Veilchen erzielt. Im Fond kann man hier zudem einen warmen-fruchtigen Charakter feststellen.

O. Parfümerie-Base Richtung Leder

| | Gewichtsteile |
|---|---|
| Styrax (natürlich) | 250 |
| Castoreum-anhydrol | 150 |
| Bergamotte-öl | 100 |
| Moschus-infusion 3% in Aethanol | 100 |
| Vetiveröl | 100 |
| Labdanum resinoid | 100 |
| Birkenteer dephenolisiert 10% in DPG | 50 |
| Ketonmoschus | 25 |
| Sandelholzöl | 10 |
| Vanillin | 10 |
| Ciste Labdanum spanisch | 5 |
| Dipropylenglykol | 60 |
| | 960 |

Mit einem Zusatz von 40 Teilen der Verbindung 3 wird sogar in obiger Lederbase ein Veilcheneffekt erzielt.

Patentansprüche

1. Verbindungen der allgemeinen Formel

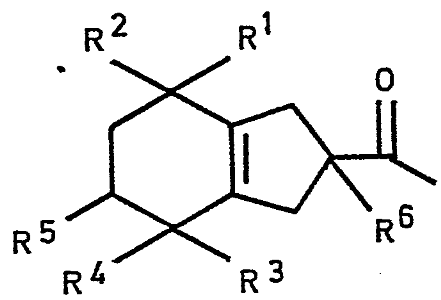

I

worin die Symbole $R^1$, $R^2$, $R^3$ und $R^4$ Wasserstoff, Methyl, Aethyl oder Isopropyl darstellen, wobei aber nicht mehr als eines dieser Symbole für Wasserstoff, Aethyl oder Isopropyl steht, und die Symbole $R^5$ und $R^6$ Wasserstoff oder Methyl darstellen.

2. 2,2,5,5-Tetramethylbicyclo[4.3.0]non-1(6)-en-8-yl-methylketon.

3. 2,2,3,5,5-Pentamethylbicyclo[4.3.0]non-1(6)-en-8-yl-methylketon.

4. 2,2-Dimethyl-5-isopropylbicyclo[4.3.0]non-1(6)-en-8-yl-methylketon.

5. 2,2,5-Trimethylbicyclo[4.3.0]non-1(6)-en-8-yl-methylketon.

6. 2,5,5-Trimethyl-2-äthylbicyclo[4.3.0]non-1(6)-8-yl-methylketon.

7. 2,2,5,5,8-Pentamethylbicyclo[4.3.0]non-1(6)-en-8-yl-methylketon.

8. Verbindungen der allgemeinen Formel

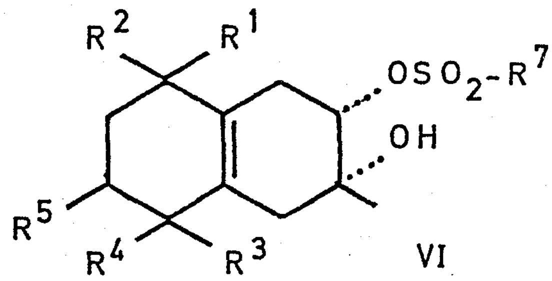

VI

worin die Symbole $R^1$, $R^2$, $R^3$ und $R^4$ Wasserstoff, Methyl, Aethyl oder Isopropyl darstellen, wobei aber jeweils nicht mehr als eines dieser Symbole für Wasserstoff, Aethyl oder Isopropyl steht, und die Symbole $R^5$ Wasserstoff oder Methyl und $R^7$ einen niederen Alkylrest oder Aryl darstellen.

9. Verbindungen der allgemeinen Formel

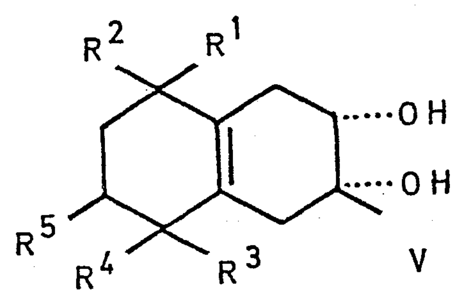

V

worin die Symbole $R^1$, $R^2$, $R^3$ und $R^4$ Wasserstoff, Methyl, Aethyl oder Isopropyl darstellen, wobei aber nicht mehr als eines dieser Symbole für Wasserstoff, Aethyl oder Isopropyl steht und $R^5$ Wasserstoff oder Methyl darstellt.

10. Verbindungen der allgemeinen Formel

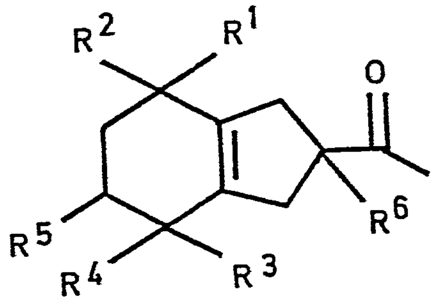

I

worin die Symbole $R^1$, $R^2$, $R^3$ und $R^4$ Wasserstoff, Methyl, Aethyl oder Isopropyl darstellen, wobei aber jeweils nicht mehr als eines dieser Symbole für Wasserstoff, Aethyl oder Isopropyl steht, und die Symbole $R^5$ und $R^6$ Wasserstoff oder Methyl darstellen,
als Riech- und/oder Geschmackstoffe.

11. Riech- und/oder Geschmackstoffkompositionen, gekennzeichnet, durch einen Gehalt an einer Verbindung der allgemeinen Formel

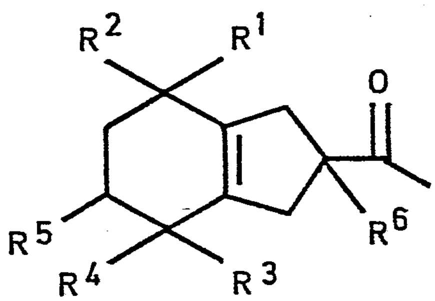

I

worin die Symbole $R^1$, $R^2$, $R^3$ und $R^4$ Wasserstoff, Methyl, Aethyl oder Isopropyl darstellen, wobei aber jeweils nicht mehr als eines dieser Symbole für Wasserstoff, Aethyl oder Isopropyl steht, und die Symbole $R^5$ und $R^6$ Wasserstoff oder Methyl darstellen.

12. Verfahren zur Herstellung der Verbindungen der allgemeinen Formel

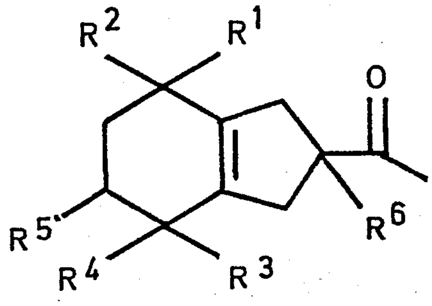

I

worin die Symbole $R^1$, $R^2$, $R^3$ und $R^4$ Wasserstoff, Methyl, Aethyl oder Isopropyl darstellen, wobei aber jeweils nicht mehr als eines dieser Symbole für Wasserstoff, Aethyl oder Isopropyl steht, und die Symbole $R^5$ und $R^6$ Wasserstoff oder Methyl darstellen,

dadurch gekennzeichnet, dass man

a) eine Verbindung der Formel

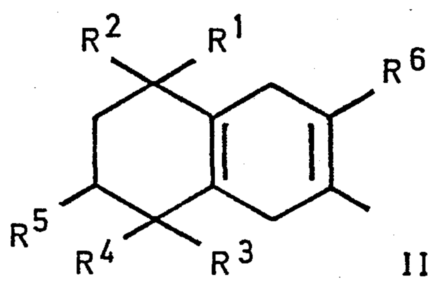

II

worin $R^1$ bis $R^5$ obige Bedeutung besitzen und $R^6$ Wasserstoff ist,

unter Ringkontraktion oxydativ umlagert, oder, dass man

b) eine Verbindung der Formel

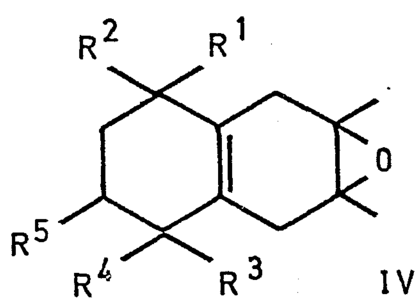

IV

worin $R^1$ bis $R^5$ obige Bedeutung besitzen, isomerisiert, oder dass man

c) eine Verbindung der Formel

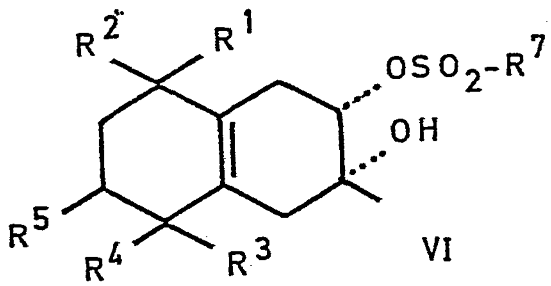

VI

worin $R^1$ bis $R^5$ obige Bedeutung besitzen und $R^7$ nieder Alkyl oder Aryl ist, unter Ringkontraktion isomerisiert.

13. Verwendung von Verbindungen der allgemeinen Formel

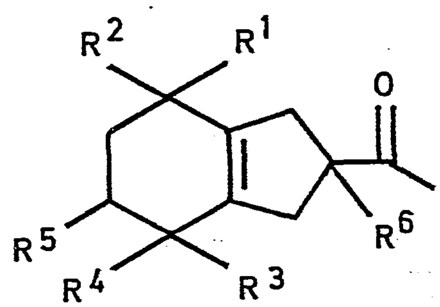

I

worin die Symbole $R^1$, $R^2$, $R^3$ und $R^4$ Wasserstoff, Methyl, Aethyl oder Isopropyl darstellen, wobei aber jeweils nicht mehr als eines dieser Symbole für Wasserstoff, Aethyl oder Isopropyl steht, und die Symbole $R^5$ und $R^6$ Wasserstoff oder Methyl darstellen, als Riech- und/oder Geschmackstoffe.

14. Verwendung von 2,2,5,5-Tetramethylbicyclo-[4.3.0]non-1(6)-en-8-yl-methylketon als Riech- und/oder Geschmackstoff.

15. Verwendung von 2,2,3,5,5-Pentamethylbicyclo-

[4.3.0]non-1(6)-en-8-yl-methylketon als Riech- und/oder Geschmackstoff.

***